# EUROPEAN PATENT SPECIFICATION

(11) **EP 0 585 251 B1**
(45) Date of publication and mention of the grant of the patent: **25.09.1996**
(21) Application number: 92909144.5
(22) Date of filing: 24.04.1992
(51) Int. Cl.: A61M 16/18

(54) **AN ANAESTHETIC VAPORISER**
NARKOSEMITTELVERDAMPFER
VAPORISATEUR ANESTHESIQUE

(30) Priority: 26.04.1991 GB 9109023
(43) Date of publication of application: 09.03.1994
(73) Proprietor: The BOC Group plc, Windlesham Surrey GU20 6HJ (GB)
(72) Inventor: KERSEY, Clifford, Graham 11 Spencer Close, Keighley West Yorkshire BD20 7DP (GB)
(74) Representative: Gough, Peter
(86) International application number: GB9200751
(87) International publication number: WO9219302

(56) References cited:
- EP-A- 0 339 828
- EP-A- 0 454 390
- GB-A- 2 097 272
- US-A- 3 528 418

## Description

This invention relates to an anaesthetic vaporiser, for supply of an anaesthetic agent to a patient.

It is common to administer anaesthetic agents supplied as liquids to a patient as a gas, by vaporising the liquid, and then carrying the vaporised liquid in a carrier gas. The conditions under which the agent is administered in this way depend on a number of factors, including the anaesthetic activity of the agent, and the quantity of agent which must be administered to a patient. The conditions can vary between extremes of rate of flow of the carrier gas.

A low flow rate of carrier gas is associated with closed breathing circuit systems where the carrier gas flow rate is determined by the metabolic gas uptake of the patient, which may be as low as 100 ml.min⁻¹, and where gas expired by the patient is returned after the carbon dioxide has been removed. The maximum concentration of anaesthetic agent vapour which can be carried by the carrier gas is typically around 30% by volume, and is determined by the saturated vapour pressure of the agent. Carrier gas saturated with agent is mixed with the expired gas in the breathing circuit to produce the required concentration of the agent for inhalation. During surgery, it can be desirable to increase the concentration of the agent in the carrier gas supplied to the patient, for example because of an increase in the uptake of agent by the patient which can cause the concentration of agent in supplied to the patient to fall, or to induce a change in the depth of anaesthesia. The limitation on the amount of the agent which can be carried by the carrier gas can be overcome by injecting liquid anaesthetic agent directly into the breathing circuit, agent vapour being picked up by the carrier gas as it flows over the liquid agent to increase the concentration in the carrier gas as required. However, it can be difficult to control accurately the variation with time of the concentration of agent which is supplied to the patient since it will depend on the rate at which agent vapour is picked up by the carrier gas. Furthermore, the injection of liquid agent into the breathing circuit can cause a rapid and undesirable increase in the amount of agent supplied to the patient in the event that some of the liquid is received by the patient as liquid, rather than as vapour in the carrier gas.

Medium and high carrier gas flow rates are associated with open and semi-closed breathing circuits, where the range of flow rates will be from about 2 to 15 l.min⁻¹. The concentration of anaesthetic agent in the carrier gas is generally in the range of 0 to 5% by volume and, under these conditions, there is little variation in the agent concentration due to factors such as an increase in the uptake of agent by the patient. However, in order to provide a steady output concentration of agent, it is necessary for large quantities of agent to be vaporised. This can be achieved by providing a chamber in which the agent is vaporised and mixed with the carrier gas. It is highly preferable that the agent be completely vaporised by the vaporiser, since supply of incompletely vaporised agent can lead to accumulation of liquid anaesthetic agent in the chamber in which the agent is vaporised and mixed with the carrier gas. This can lead in turn to significant fluctuations in the rate at which the agent is administered to a patient: as the agent collects, the effective amount of agent administered to the patient is less than intended; subsequently, as that agent vaporises, the effective amount of agent administered to the patient is greater than intended. Furthermore, the collection of liquid agent makes flushing of agent out of the apparatus more difficult.

The use of a chamber in which the agent is vaporised and mixed with the carrier gas cannot be used when the flow rate of the carrier gas is low, for example less than about 0.8 l.min⁻¹, since it exacerbates the problem associated with low flow rate regimes of controlling changes in agent concentrations accurately.

It is preferred that a single machine be used to administer an anaesthetic agent to a patient in a carrier gas, whether the flow rate of the carrier gas is low or high, but the differing requirements of these two regimes make this difficult. US-4611590 discloses apparatus for adding a liquid anaesthetic agent to respiratory gas supplied to a patient, in which the agent is supplied to a chamber in which it is heated to cause vaporisation, for subsequent supply directly to a patient's breathing circuit. Alternatively, the agent can be supplied to a swirl chamber where it is mixed with a carrier gas. From the swirl chamber the agent with its associated carrier gas is administered directly to a patient's breathing circuit. The apparatus therefore provides the possibility either of administering the anaesthetic agent carried by a large volume of a carrier gas, or directly without any carrier gas. However, the disclosed apparatus requires the use of the combination of a heated chamber, a temperature sensor and heated conduit to operate in this way.

US Patent 3528418 discloses an anaesthetic vaporiser which includes a conduit for carrier gas, for example oxygen, in which is located a controller for regulating the pressure of the carrier gas in the conduit. The conduit extends from the controller to a gas distributor which is submerged in liquid anaesthetic agent contained within a vessel. In use, a mixture of entrained anaesthetic agent and carrier gas leaves the vessel through a delivery conduit for administration to a patient.

A first by-pass line is connected to the conduit for carrier gas for supplying pure oxygen to the patient.

A second by-pass line is connected to the conduit for carrier gas and the pressure of carrier gas in this second by-pass line is regulated by a valve which is set to ensure oxygen will only flow through the second by-pass line when at a pressure above a predetermined minimum.

The present invention provides appartus for supply of an anaesthetic agent in which liquid agent is mixed initially with a small quantity of a carrier gas, for subsequent administration to a patient, possibly with just that small quantity of carrier gas, or alternatively after mixing with a larger quantity of carrier gas.

According to the present invention, an anaesthetic vaporiser comprises:
a) a unit for supplying carrier gas with a composition selected according to the requirements of a patient to whom it is to be supplied from the vaporiser;
b) a regulator by which the quantity of the carrier gas flowing from the supply unit into a first passageway is controlled so that no more than a pre-determined quantity of gas flows through the passageway;
c) a reservoir for an anaesthetic agent, from which the agent is supplied into said first passageway to mix with the carrier gas flowing therein, for subsequent administration to a patient;
d) a second passageway for the carrier gas which extends to a point at which the first and second passageways join to allow the anaesthetic agent and carrier gas in the first passageway to mix with carrier gas in the second passageway prior to administration of the anaesthetic agent to a patient;
e) a first sub-passageway which is connected to the second passageway for administration to a patient of carrier gas in the first and second passageways together with any anaesthetic agent in the first passageway after mixing of the said carrier gas and anaesthetic agent;
   characterised in that the second passageway for the carrier gas extends from the regulator to the point at which the first and second passageways join, and in that, a second sub-passageway is provided by which carrier gas together with any anaesthetic agent in the first passageway can be administered directly to a patient without prior mixing with the carrier gas in the second passageway; and means for selecting flow of carrier gas in the first passageway and any anaesthetic agent carried with it between the first and second sub-passageways.

The apparatus of the invention has the advantage that a greater degree of control over the anaesthetic agent supplied to a patient is available whether the anaesthetic agent is being supplied directly after vaporisation or atomisation to the patient's breathing circuit, or it is being supplied after mixture with an additional quantity of a carrier gas.

The carrier gas supply unit will generally include an adaptor which can accommodate a number of separate cases provided in separate containers, generally cylinders which hold gases under pressure. The supply unit will include appropriate control equipment by which the mixture of the carrier gas which is supplied from it is selected according to the, requirements of a patient to whom the carrier gas, together with any associated anaesthetic agent, is to be supplied. It will therefore be understood that the selection of a carrier gas formulation is carried out in the present vaporiser prior to the addition to it of anaesthetic agent and, indeed, to the split of the carrier gas between the first passageway and the second passageway.

The mechanism by which the carrier gas in the first passageway and agent which is supplied to it mix can involve atomisation of liquid agent, evaporation or a combination of the two. For example, a liquid agent maybe broken into small droplets by passing it with a carrier gas through an orifice. Subsequently, small droplets of liquid agent may evaporate between the orifice and entry into the patient's breathing circuit. Indeed, in some applications, the evaporation of agent can take place after entry into the breathing circuit. This is particularly applicable when the rate of addition of agent to the breathing circuit is relatively high, but the rate of flow of carrier gas is low. The apparatus of the invention may include additional components by which vaporisation or atomisation of the liquid agent is encouraged. For example, a heat source or a metal coil (especially a copper coil) may be included to encourage evaporation. Alternatively, or in addition, a member may be provided with a component which defines an orifice through which the carrier gas and any agent flowing in the first passageway must pass, so that atomisation results from the change in pressure as the gas and agent pass out of the orifice. The member may be, for example, a plate having a hole in it, or a length of a tube having an internal bore which is smaller than that of the passageway. After passage through such an orifice, liquid agent in the passageway will generally be broken into small droplets.

The apparatus of the invention has the advantage that, when arranged to supply anaesthetic agent directly to a patient's breathing circuit with a low carrier gas flow rate without the agent first being mixed with an additional quantity of a carrier gas, the concentration of the agent in the breathing circuit can be adjusted quickly and conveniently, and is not dependent on the degree of saturation of carrier gas, as can be the case in vaporisers in which carrier gas is passed through a quantity of liquid anaesthetic agent. This is made possible because of the provision of a small quantity of carrier gas for vaporising or atomising the agent after discharge from the reservoir. Furthermore, the ability to supply an agent directly to a patient's breathing circuit after vaporisation or atomisation has the significant advantage that the response time for a change in concentration of the agent can be kept low. In these respects, the apparatus of the invention is to be contrasted with systems in which anaesthetic agent is supplied as liquid directly to a patient's breathing circuit to be picked up by carrier gas flowing over it, where the agent concentration in the carrier gas is dependent on the rate at which the agent is picked up by the gas, which is itself dependent on factors such as the degree of saturation and temperature of the gas.

The apparatus of the invention has the further significant advantage that it is able to provide an anaesthetic agent directly following atomisation or vaporisation, or following mixing with an additional quantity of carrier gas, particularly simply, for example without the need for a heated chamber, a temperature sensor and a heated conduit as is called for in the apparatus disclosed in US-4611590.

Anaesthetic agent is preferably added to the carrier gas flowing in the first passageway in such a way that the quantity that is added is controlled continuously and carefully. This may be achieved by means of a dosing pump. It has been found possible by use of such a pump to control the rate of supply of the anaesthetic agent in the range of from 2 µ1.min⁻¹ to 11000 µ1.min⁻¹. For example, the pump can be made to deliver anaesthetic agent at rates which vary by a factor of as much as 5500. For example, the rate of flow of carrier gas through the vaporiser might vary between 0.2 and 15 litres per minute, and the anaesthetic concentration might vary between 0.2 and 12% by volume.

The apparatus of the invention may be used to provide anaesthetic agent in a carrier gas which has a high flow rate. In this arrangement, the carrier gas in the first passageway, together with any agent associated with it, is mixed with additional carrier gas flowing in the second passageway, to increase the total amount of gas in which the agent is carried for administration to a patient. This can be an attractive arrangement when the quantity of agent or carrier gas or both to be administered to a patient is high.

The split of carrier gas between the first and second passageways, by the regulator, is arranged so that no more than a predetermined quantity of gas flows through the first passageway, and so that the excess gas flows through a second passageway. The regulator can ensure that only sufficient gas flows through the first passageway to ensure adequate carriage of the anaesthetic agent. The quantity of gas which is allowed by the regulator to flow through the first passageway will be selected to ensure an appropriate degree of atomisation or vaporisation or both of liquid agent to be mixed with it.

The regulator is preferably a valve which opens when the pressure exerted against it exceeds a pre-determined minimum pressure. Such valves are commonly referred to as back-pressure valves. An appropriate valve might comprise, for example, an aperture with an appropriate closure member which is biassed against the aperture so as to close it, for example by means of a spring. The closure member may be, for example, a ball which acts against a circular aperture in a plate under force exerted by a spring.

Preferably, the regulator will allow for the quantity of gas to be selected. For example, the regulator may be set to ensure that the maximum rate of flow of gas through the first passageway is about 1 litre per minute. It has been found that an appropriate back-pressure for many applications is less than about 7.5 x 10³ kg.m⁻², preferably less than about 5 x 10³ kg.m⁻². The back-pressure may be greater than about 1.5 x 10³ kg.m⁻², preferably greater than about 2.5 x 10³ kg.m⁻².

The first and second passageways are connected to one another so that the carrier gas in the passageways, together with any anaesthetic agent in the first passageway mixed with the gas, can mix prior to administration to a patient. Mixing of the carrier gas and agent in the passageways may take place in a chamber, which is configured to ensure proper mixing of the agent vapour and the carrier gas, and full vaporisation of the agent. Preferably, the carrier gas and anaesthetic agent mixed with it enter the chamber through a nozzle, which maintains a pressure difference between the first passageway and the mixing chamber and second passageway. The nozzle diameter will be selected according to the pressures involved. A nozzle diameter of less than about 1.0 mm, for example about 0.5 mm, has been found to be appropriate for many applications.

The selection means may comprise a simple switch by which the flow of gas can be selected. The provision of selection means of this type has the advantage that the adaptability of the apparatus of the invention between high and low gas flow administration conditions can be exploited to the fullest possible extent.

The size of the second sub-passageway, through which carrier gas and associated anaesthetic agent are administered directly to a patient, will generally be selected according to the volumes of fluid to be administered, while maintaining an appropriate pressure in the first passageway. The size of the second sub-passageway can usefully be less than that of the first passageway. It can be preferred for some applications for the second sub-passageway to have an internal diameter of less than about 5 mm, more preferably less than about 3.5 mm, for example about 2 mm.

The passageways or sub-passageways through which carrier gas and anaesthetic agent are administered to a patient are preferably provided with appropriate fittings so that they can be interfaced appropriately with the patient's breathing circuit.

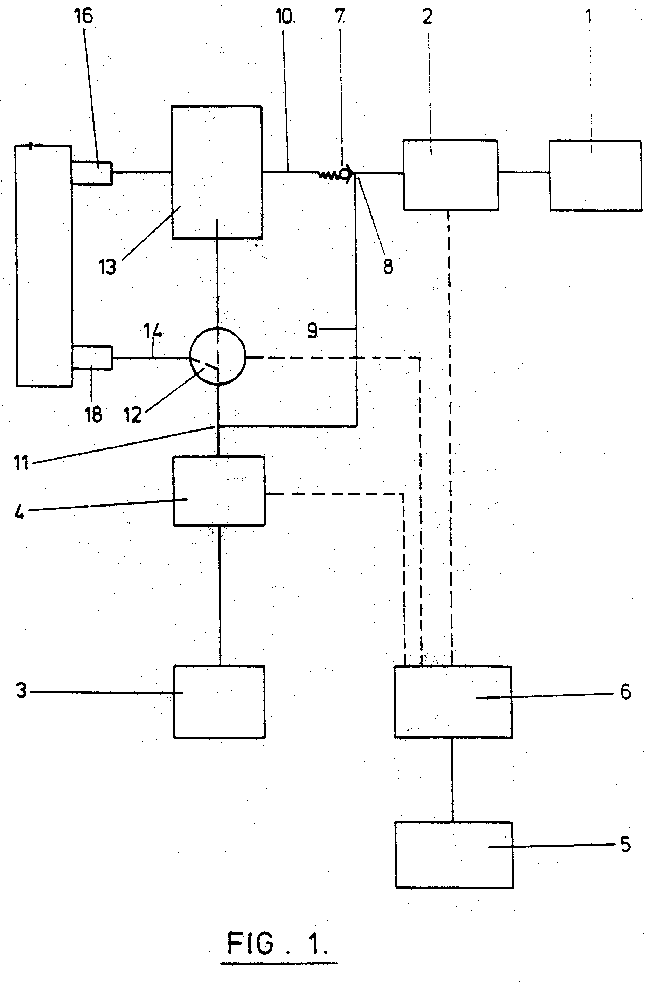

Embodiments of the present invention will now be described by way of example with reference to the accompanying drawing, which provides a schematic illustration of the apparatus of the invention.

Referring to the drawings, the apparatus comprises a source 1 of a carrier gas, which might consist of, for example nitrous oxide, oxygen or air, or a combination of these gases. The carrier gas flows from the source 1 through a flow sensor 2, which allows the rate of flow of carrier gas to be monitored.

A back pressure regulator 7 is situated downstream of the flow sensor 2 to control the split of carrier gas at a junction 8 between a first passageway 9 and a second passageway 10. The regulator 7 ensures that no more than a predetermined quantity of gas flows through the first passageway 9, excess gas being admitted to the second passageway 10. Liquid anaesthetic agent is held in a reservoir 3, and is supplied to the first passageway 9 through a dosing pump 4 by which the rate of supply of the anaesthetic agent is controlled, according to the required concentration of agent and the required rate of flow of gas into the patient's breathing circuit.

Anaesthetic agent supplied from the reservoir 3 enters the first passageway at a junction 11. The mixture of carrier gas and agent then flows to a switch 12 by which the subsequent flow of the gas and agent can be selected between a first sub-passageway which conducts the gas and liquid to a mixing chamber, and a second sub-passageway 14 through which the agent and carrier gas are administered to the breathing system of a patient, via an outlet 18. The first sub-passageway conducts the carrier gas and agent to a mixing chamber 13 through a nozzle which ensures atomisation of the liquid agent, where it is diluted with excess gas flow which flowed from the regulator 7 through the second passageway 10, leading also to the mixing chamber. A third passageway leads directly from the mixing chamber to an outlet 16, through which carrier gas from the first and second passageways, and agent, can be administered to the breathing system of a patient.

The apparatus includes control equipment 5, 6 for the supply unit and the dosing pump 4.

The apparatus of the invention may include more than one reservoir, so that the apparatus can be used to supply a number of different anaesthetic agents by appropriate selection of the reservoir from which an agent is to be supplied to the first passageway. Different anaesthetic agents can require administration to a patient under different conditions, and the apparatus of the invention allows the conditions necessary for administration of each specific agent to be attained conveniently.

## Claims

1. An anaesthetic vaporiser which comprises:
a) a unit (1) for supplying carrier gas with a composition selected according to the requirements of a patient to whom it is to be supplied from the vaporiser;
b) a regulator (7) by which the quantity of the carrier gas flowing from the supply unit (1) into a first passageway (9) is controlled so that no more than a pre-determined quantity of gas flows through the passageway (9);
c) a reservoir (3) for an anaesthetic agent, from which the agent is supplied into said first passageway (9) to mix with the carrier gas flowing therein, for subsequent administration to a patient;
d) a second passageway (10) for the carrier gas which extends to a point at which the first and second passageways join to allow the anaesthetic agent and carrier gas in the first passageway (9) to mix with carrier gas in the second passageway (10) prior to administration of the anaesthetic agent to a patient;
e) a first sub-passageway which is connected to the second passageway (10) for administration to a patient of carrier gas in the first and second passageways (9,10) together with any anaesthetic agent in the first passageway (9) after mixing of the said carrier gas and anaesthetic agent;
characterised in that the second passageway (10) for the carrier gas extends from the regulator (7) to the point at which the first and second passageways (9, 10) join, and in that, a second sub-passageway (14) is provided by which carrier gas together with any anaesthetic agent in the first passageway (9) can be administered directly to a patient without prior mixing with the carrier gas in the second passageway (10); and means (12) for selecting flow of carrier gas in the first passageway and any anaesthetic agent carried with it between the first and second sub-passageways.

2. A vaporiser as claimed in claim 1, which includes a mixing chamber (13) in which the carrier gas in the first and second passageways (9, 10) can mix.

3. A vaporiser as claimed in claim 1 or 2, which includes a source of heat by which vaporisation of anaesthetic agent can be encouraged.

4. A vaporiser as claimed in any one of claims 1 to 3, which includes a component which defines an orifice through which the carrier gas and any anaesthetic agent carried with it must pass to cause atomisation of the anaesthetic agent.

5. A vaporiser as claimed in any one of claims 1 to 4, in which the regulator (7) is a back pressure valve.

## Patentansprüche

1. Narkosemittelverdampfer, der aufweist:
a) eine Einheit (1) zum Zuführen von Trägergas mit einer Zusammensetzung, die entsprechend der Bedürfnisse eines Patienten gewählt ist, zu welchem es aus dem Verdampfer zugeführt wird,
b) einen Regler (7), mit welchem die Menge des aus der Zufuhreinheit (1) in eine erste Leitung (9) einströmenden Trägergases so gesteuert wird, daß nicht mehr als eine vorgegebene Gasmenge durch die Leitung (9) strömt,
c) einen Behälter (3) für ein Narkosemittel, aus welchem das Mittel in die erste Leitung (9) zugeführt wird, um sich mit den darin strömenden Trägergas zu vermischen, für die nachfolgende Verabreichung an einem Patienten,
d) eine zweite Leitung (10) für das Trägergas, die zu einer Stelle verläuft, an welcher die erste und die zweite Leitung sich miteinander vereinigen, damit sich das Narkosemittel und das Trägergas in der ersten Leitung (9) mit Trägergas in der zweiten Leitung (10) vor der Verabreichung des Narkosemittels an einem Patienten vermischen kann,
e) einen ersten Leitungszweig, der mit der zweiten Leitung (10) verbunden ist, um Trägergas in der ersten und der zweiten Leitung (9, 10) zusammen mit Narkosemittel in der ersten Leitung (9) nach Vermischung des Trägergases und des Narkosemittels zu einem Patienten zu verabreichen,
dadurch gekennzeichnet, daß die zweite Leitung (10) für das Trägergas vom Regler (7) zu der Stelle verläuft, an welcher sich die erste und die zweite Leitung (9, 10) vereinigen, und daß ein zweiter Leitungszweig (14) vorgesehen ist, durch welchen Trägergas zusammen mit Narkosemittel in der ersten Leitung (9) ohne vorherige Vermischung mit dem Trägergas in der zweiten Leitung (10) direkt an einem Patienten verabreicht werden kann, und daß Mittel (12) zum wahlweisen Umschalten der Trägergasströmung in der ersten Leitung und damit mitgeführten Narkosemittel zwischen dem ersten und dem zweiten Leitungszweig vorgesehen sind.

2. Verdampfer nach Anspruch 1, der eine Mischkammer (13) aufweist, in welcher das Trägergas in der ersten und der zweiten Leitung (9, 10) sich vermischen kann.

3. Verdampfer nach Anspruch 1 oder 2, der eine Wärmequelle aufweist, mittels welcher die Verdampfung des Narkosemittels unterstützt werden kann.

4. Verdampfer nach einem der Ansprüche 1 bis 3, der ein Bauteil aufweist, das eine Düse bildet, durch welche Trägergas und mitgeführtes Narkosemittel hindurchpassieren müssen, um eine Zerstäubung des Narkosemittels zu bewirken.

5. Verdampfer nach einem der Ansprüche 1 bis 4, bei welchem der Regler (7) ein Gegendruckventil ist.

## Revendications

1. Vaporisateur d'anesthésique qui comprend :
(a) une unité (1) pour fournir un gaz porteur avec une composition choisie en fonction des exigences propres à un patient auquel il doit être alimenté à partir du vaporisateur;
(b) un régulateur (7) grâce auquel la quantité de gaz porteur s'écoulant de l'unité d'alimentation (1) dans un premier passage (9) est contrôlée de façon que seule une quantité prédéterminée de gaz s'écoule dans le passage (9) ;
(c) un réservoir (3) pour un agent anesthésique, à partir duquel l'agent est alimenté dans ledit premier passage (9) pour se mélanger au gaz porteur s'y écoulant, pour une administration ultérieure à un patient;
(d) un second passage (10) pour le gaz porteur qui s'étend jusqu'à un point où le premier et le second passages se rejoignent pour permettre à l'agent anesthésique et au gaz porteur dans le premier passage (9) de se mélanger au gaz porteur dans le second passage (10) avant l'administration de l'agent anesthésique à un patient;
(e) un premier passage secondaire qui est raccordé au second passage (10) pour l'administration à un patient du gaz porteur dans les premier et second passages (9, 10) en même temps que tout agent anesthésique dans le premier passage (9) après mélange desdits gaz porteur et agent anesthésique ;
***caractérisé en ce que*** le second passage (10) pour le gaz porteur s'étend du régulateur (7) au point où le premier et le second passages (9, 10) se rejoignent, ***et en ce qu'***un second passage secondaire (14) est prévu, par lequel le gaz porteur avec tout agent anesthésique dans le premier passage (9) peuvent être administrés directement à un patient sans mélange préalable avec le gaz porteur dans le second passage (10); et des moyens (12) pour choisir l'écoulement de gaz porteur dans le premier passage et de tout agent anesthésique transporté avec lui entre le premier et le second passages secondaires.

2. Vaporisateur selon la Revendication 1, qui comprend une chambre de mélange (13) dans laquelle peut se mélanger le gaz porteur des premier et second passages (9, 10).

3. Vaporisateur selon la Revendication 1 ou 2, qui comprend une source de chaleur grâce à laquelle la vaporisation de l'agent anesthésique peut être favorisée.

4. Vaporisateur selon l'une quelconque des Revendications 1 à 3, qui comprend un composant qui définit un orifice au travers duquel le gaz porteur et tout agent anesthésique porté par lui doivent passer pour provoquer l'atomisation de l'agent anesthésique.

5. Vaporisateur selon l'une quelconque des Revendications 1 à 4, dans lequel le régulateur (7) est une soupape de retenue.
